# EUROPÄISCHE PATENTSCHRIFT

(11) **EP 0 396 560 B1**
(45) Veröffentlichungstag und Bekanntmachung des Hinweises auf die Patenterteilung: **02.11.1994**
(21) Anmeldenummer: 88909728.3
(22) Anmeldetag: 18.11.1988
(51) Int. Cl.: A61K 39/00, A23K 1/16, A23K 1/18, A23L 1/30

(54) **VERFAHREN ZUR STEIGERUNG VON WACHSTUMSGESCHWINDIGKEIT UND/ODER NUTZUNG VON FUTTER- ODER NAHRUNGSMITTELN UND/ODER RESISTENZ BEI TIEREN UND MENSCHEN**
PROCESS FOR INCREASING THE GROWTH RATE AND/OR UTILIZATION OF FODDER OR FOODSTUFFS AND/OR RESISTANCE IN ANIMALS AND HUMANS
PROCEDES PERMETTANT D'AUGMENTER LE TAUX DE CROISSANCE ET/OU LE RENDEMENT DES FOURRAGES ET PRODUITS ALIMENTAIRES ET/OU LA RESISTANCE CHEZ LES ANIMAUX ET L'HOMME

(43) Veröffentlichungstag der Anmeldung: 14.11.1990
(73) Patentinhaber: EFFEM GmbH, D-27283 Verden (DE)
(72) Erfinder: Teichmann, Reinhard, Prof. Dr., W-7417 Pfullingen (DE); Liebich, Hans-Georg, Prof. Dr., W-8000 München 40 (DE)
(74) Vertreter: Goddar, Heinz J., Dr.
(86) Internationale Anmeldenummer: DE8800724
(87) Internationale Veröffentlichungsnummer: WO9005536

(56) Entgegenhaltungen:
- EP-A- 219 979
- EP-A- 286 847
- WO-A-86/03409
- WO-A-86/07539
- WO-A-87/04050
- WO-A-88/08699
- GB-A- 2 008 404
- GB-A- 2 132 466
- THE VETERINARY RECORD, Band 97, Nr. 2, 12 Juli 1975, GB; P. PORTER et al., Seiten 24-28#

## Beschreibung

Die Erfindung betrifft Verfahren zur Steigerung von Wachstumgeschwindigkeit und/oder Nutzung von Futtermitteln und/oder Resistenz bei Tieren, sowie die Verwendung von mindestens einem Antigen zum Gebrauch in einem solchen Verfahren bzw. zur Herstellung eines Medikamentes zum Einsatz bei einem solchen Verfahren.

Die seit vielen Jahrzehnten eingesetzten Futtermittel bzw. Futtermittelzusatzstoffe und andere Verfahren zur Förderung des Wachstrums der Tiere und zur Verbesserung der Nährstoffnutzung zeigen nur eingeschränkt Erfolg.

Antibiotika in Futtermitteln führen nicht nur zu irreparablen Gesundheitsschäden der Tiere durch zunehmende Bildung von resistenten Erreger-Stämmen mit der Gefahr von Sekundärerkrankungen, sondern erweisen sich auch für den Menschen als Endverbraucher der Lebensmittel tierischen Ursprungs (z.B. Fleisch, Milch, Butter, Eier) als höchst gesundheitsgefährdend.

Hormone schädigen noch stärker u.a. aufgrund ihrer anabolen Förderung der Wachstumsgeschwindigkeit die Gesundheit von Tier und Mensch.

Die genannten Wirkstoffe können zudem nicht und gewünschten Maß wieder aus dem Körper der Tiere ausgeschieden werden, d.h. sie kumulieren im Tier als Rückstände bei Fleisch, Milch, Butter und Eiern.

Die weitweite Verwendung der genannten Wirkstoffe führte in den Industrieländern zur Überproduktion minderwertiger Lebensmittel tierischen Ursprungs, deren Genuß den Menschen, besonders stark gesundheitlich gefährdet.

Da die herkömmlichen Erzeugnisse und Verfahren diese zahlreichen gesundheitsschädlichen Nachteile aufweisen und keine geeigneten anderen Verfahren im Zusammenhang mit den kommerziell verwendeten Futtermitteln bekannt sind, besteht seit vielen Jahrzehnten ein dringendes Bedürfnis an neuartigen Erzeugnissen bzw. Verfahren.

Der jährliche Gesamtproduktionswert der Landwirtschaft allein in Deutschland übersteigt 60 Mrd. DM bei folgenden Anteilen:
- tierische Produktion über 40 Mrd. DM, davon
- Rinder über 25 Mrd. DM und
- Schweine über 11 Mrd. DM
(Horst Kräußlich, Neue Entwicklungen auf dem Gebiet der Tierzucht und deren wirtschaftliche Bedeutung, GRUR 1987, 340).

Aus der GB-A-2008404 ist ein Verfahren zur Steigerung der Wachstumsgeschwindigkeit bei Geflügel, das mit einem Futter großgezogen wird, das zugesetzte, lebensfähige, sporulierte Oozysten wenigstens einer Kokzidienart enthält, für die das Geflügel anfällig ist, wobei die Oozysten in einer Konzentration vorhanden sind, die nur ausreichend ist, um eine subklinische Infektion im Geflügel zu induzieren.

Aus der WO-A-88/08699 ist die Verwendung einer Zusammensetzung zur oralen Immunisierung und dadurch bedingten Wachstumssteigerungen von Tieren, wie etwa Geflügel bekannt, die feste Teilchen einer Lipid-kontinuierlichen Emulsion von wenigstens 1 gw.-%, aber nicht mehr als 70 gw.-% in Lipid mit einem Schmelzpunkt im Bereich von 30 bis 55°C umfaßt, wobei die Zusammensetzung pro Teilchen im Mittel wenigstens ein lebendes, abgekapseltes, parasitisches Protozoon enthält.

Aus The Veterinary Record 92,630,636 (1973) zur Wachstums- und Resistenzsteigerung bei Schweinen durch orale Immunisierung und weitere orale Gabe durch Hitze-inaktivierten Antigen-Zubereitungen von E. Coli-Stämmen, welche zu Darmkrankheiten führen.

Aus The Veterinary Record 97,24-28 (1975) ist schließlich ein Verfahren zur Wachstums- und Resistenzsteigerung bei präruminanten Kälbern durch orale Immunisierung und weitere orale Gabe durch Hitze-inaktivierten Antigen-Zubereitungen von E. Coli- und Salmonella-stämmen welche zu Darmkrankheiten führen, bekannt.

EP-A-0 286 847 beschreibt das anmeldungsgemäße Verfahren ober für einen anderen Zweck und zwar zur Ulkusprotektion und Behandlung von Karzinomen, Erosionen im Verdauungstrakt und Durchfällen.

**Aufgabe** der Erfindung ist daher, Erzeugnisse und Verfahren zur Steigerung von Wachstumsgeschwindigkeit bzw. Nutzung von Futter- und Nahrungsmitteln bzw. Resistenz bei Tieren und Menschen anzugeben, die biologisch wirksam und (daher) ohne jegliche Rückstandsprobleme sind.

Der Lösung der Aufgabe liegt die **Erkenntnis** eines immun-biologischen Mechanismus zugrunde, wie durch Untersuchungen der Erfinder an Labortieren (Hunden und Ratten) und Mast-Schweinen bewiesen.

Die erfindungsgemäße **Lösung** der Aufgabe erfolgt durch die Lehre der Ansprüche 1, 10, 11.

Vorteilhafte Ausgestaltungen der Erfindung enthalten die übrigen Ansprüche.

Die erfindungsgemäße Steigerung von
Wachstumsgeschwindigkeit bzw. Nutzung der Futter- bzw. Nahrungsmittel beträgt leicht errreichbar mindestens einige 10 %,
wie von den Erfindern insbesondere in der Schweinemast (vgl. weiter unten Versuche 1 und 2) nachgewiesen anhand von dort
um 30,4 % gesteigerter Futtermittelnutzung.

### Wissenschaftliche Erkenntnisse

Sekretion und damit Verdauung des Magens werden üblicherweise in die kephale, gastrale und intestinale Phase eingeteilt.

Säure- und Pepsin-Sekretion werden dabei am stärksten in der gastralen Phase stimuliert.

Während dieser Phase wird das antrale Hormon Gastrin als der wichtigste Stimulus für die Säure-Sekretion freigesetzt ¹. Die Säure-Sekretion ist notwendig zur Denaturierung der Proteine und ihren Abbau und auch die erste Infektionsbarriere. Die Säure, die über den Pylorus in den Bulbus duodeni gelangt, führt zur Freisetzung von Sekretin und auch in geringem Umfang Cholezystokinin ².

Sekretin stimuliert stark die wäßrigen Komponenten der Pankreas-Sekretion und spült so die Pankreas-Enzyme heraus.

Durch diese Mechanismen wird die gesamte Kaskade der Verdauung eingeleitet.

Ein Schlüsselhormon in der gastralen Phase der Verdauung und folglich für die Induktion der Verdauungsprozesse im gastrointestinalen Trakt ist das Gastrin. Obwohl es über neurogene, mechanische und hormonale Stimuli freigesetzt wird, ist diese Freisetzung entscheidend jedoch von den einzelnen Nahrungsbestandteilen abhängig, die in den Magen gelangen. Proteine und Peptide sind die wesentlichen Substanzen in der Nahrung, die Gastrin freisetzen.

Die Erfindung geht dabei von der Erkenntnis aus, daß mit jeder Mahlzeit die Schleimhaut (Mukosa) des Magens in Kontakt mit Substanzen kommt, die im Magen-Darm-Trakt als Antigene immun-biologisch wirksam werden könnten.

### Untersuchungen an Hunden

Die Erfinder haben entgegen der seit vielen Jahren gängigen Meinung ¹ bei Hunden insbesondere folgendes festgestellt:
Bei nicht-immunisierten Hunden (20 Bastard-Hunden)
rief die Gabe in, den Magen eines Antigens, nämlich humanes Gammaglobulin (HGG) als ein dem Hund fremdes Eiweiß, keine hormonale Stimulation hervor.

In immunisierten Hunden dagegen
kam es zu hoch-signifikanter und starker Freisetzung von Serumgastrin, gleichzeitig zu signifikanter Zunahme der Durchblutung in der Mukosa des Antrums, des Bulbus duodeni und der Duodenalmukosa nach 10 min.

Nach intragastraler Gabe von menschlichem Albumin als nicht-antigenem Protein zeigte sich keine Freisetzung von Gastrin, was die Spezifität der immunologischen Reaktion beweist.

Wenn der Grad der Immunisierung abnahm, etwa 8 Wochen nach der letzten Boosterung, erfolgte nach Gabe von HGG in den Hundemagen nur noch eine geringfügige Freisetzung von Gastrin. Reimmunisierung führte wieder zu signifikantem Gastrin-Anstieg.

Zusätzlich konnte gezeigt werden, daß die Gabe von kommerziellem Hundefutter vor und nach der Immunisierung mit HGG keinerlei Änderungen in der Gastrinfreisetzung ergab.

Die Gastrin-Freisetzung wurde im weiteren verglichen mit derjenigen nach bekannter Proteinstimulation wie kommerzieller Formular-Diät zur enteralen Ernährung von Menschen (Biosorbin^{R}) mit 5 g Proteinanteil, aufgelöst im gleichen Volumen. Dabei ergab sich fast keine Hormonfreisetzung, obwohl der Anteil an Proteinen und Peptiden, berechnet auf molarer Basis, größer im Biosorbin^{R} ist als in der HGG-Lösung.

Andererseits führte die Gabe von 5 g bovinem Serum Albumin (BSA), einem dem Hund bekannten Protein, zu signifikanter Stimulation entsprechend der nach Hundefutter.

Auch die Sekretin-Freisetzung wurde verglichen.

Es kam zu starker Stimulation des Sekretins nach Gabe von 1 g HGG bei immunisierten Tieren, genau so stark, wie nach einer Futterstimulation.

Nach Gabe von BSA kam es allerdings zu keiner Sekretin-Freisetzung, wahrscheinlich bedingt durch die hohe Pufferkapazität dieser Substanz.

### Cardiovasculäre und respiratorische Parameter:

Es kam zu keinerlei signifikanten Veränderungen des Herz-Minuten-Volumens, des Blutdrucks und der Blutgase bei beiden Gruppen von Tieren während der Studie, d.h. weder in immunisierten noch in nicht-immunisierten Tieren mit und ohne Antigengabe.

Es wurden keinerlei Einflüsse auf die respiratorischen Funktionen beobachtet.

### Untersuchungen an Ratten

Das erfindungsgemäß erkannte Phänomen der immunologischen Stimulation der Verdauung wurde auch bei Ratten von den Erfindern nachgewiesen.

### Anwendung der Untersuchungen

Die verbesserte Verdauung und Verstoffwechselung des Eiweißes, das als Antigen benutzt wurde (und aller Eiweiße im Futter), hängt von den Bauchspeicheldrüsen-Enzymen ab.

Dieses Phänomen läßt sich dadurch erklären, daß die gesamte gastroenteropankreatische Kaskade immunologisch stimuliert werden kann:
- Es beginnt mit der Gastrin-Freisetzung,
- gefolgt von der Säure- und Schleim-Produktion,
- die schließlich zu einer Sekretin-Freisetzung mit allen ihren Effekten führt.

Die Entdeckung des immunologischen Mechanismus zur Stimulation der Verdauungsfunktionen bedeutet einen neuen wichtigen Beitrag, um wirksam die Verdauung zu steuern.

Dieser Mechanismus bedient sich physiologischer Prozesse;
er ist "biologischer",
seine Wirksamkeit wurde in Feldversuchen (vgl. weiter unten) unter ökonomisch relevanten Bedingungen nachgewiesen.

Eine verbesserte Futter- oder Nahrungs-Nutzung ist von größter Wichtigkeit für Tier und Mensch mit ähnlich funktionierendem gastrointestinalen System, das ähnlich auf diesen erfindungsgemäß erkannten immunologischen Mechanismus reagiert. Die gleichartige Reaktion in Hund, Ratte und Schwein (vgl. unten) beweist dies.

Das herausragende Merkmal des entdeckten, der Erfindung zugrundegelegten, Mechanismus ist die stark gesteigerte Verdauungsleistung durch Induktion von vor allem Magen- und insbesondere Bauchspeicheldrüsen-Sekretion:

Gelangen in den Magen geringe Mengen des Antigens, gegen das das Tier immunisiert worden war, stellen diese einen sehr starken Stimulus gastrointestinaler Funktionen dar. Gastrin und Sekretin werden im folgenden freigesetzt, Magen- und Bauchspeicheldrüsen-Sekretion steigen an. Diese Vorgänge zeigen, daß geringe Mengen z.B. eines Proteins bei dagegen immunisierten Tieren ein starkes Ansteigen der Verdauungsleistung hervorrufen.

Für die Stimulation der Verdauung über diese immunologischen Mechanismen ist nicht die Menge der aufgenommenen Nahrung entscheidend, sondern die Zusammensetzung der Nahrung: Die Nahrung muß z.B. das Protein enthalten, das der Magen immunologisch erkennen kann. Dieser immunologische Stimulus ist sogar größer als der durch Vagusnerv, Hormone oder Dehnung des Magens ausgelöste. Diese Erkenntnisse der Erfinder über den immunologischen Mechanismus sind die wissenschaftliche Grundlage der Wachstumssteigerung und der Verbesserung der Futter- bzw. Nahrungsmittelnutzung

Die Behandlung beinhaltet folglich die Verwendung einer natürlich vorkommenden oder einer synthetischen Substanz, die entweder bereits im Futter oder in der Nahrung des Menschen vorhanden ist oder zugesetzt werden kann; vorausgesetzt, daß das Tier oder der Mensch gegen diese spezielle Substanz sensibilisiert wird. Eine orale Sensibilisierung geschieht vorwiegend nur beim Neugeborenen oder beim Frühgeborenen. Die Behandlung besteht nun darin, daß eine biologische (d.h. natürlich vorkommende) oder synthetische Substanz (oder Hapten), gegen die der Organismus immunisiert wird, im Futter oder in der Nahrung, im Trinkwasser oder anderen Flüssigkeiten als feste oder flüssige Form als Tablette, Bolus als Granula oder in Säften, Sirup oder Melasse oder Soßen gegeben wird. Zusätzlich kann die Gabe allein oder in Kombination mit anderen bereits geprüften oder generell als allgemein sicher anerkannten Substanzen oder Nahrungsmitteln, die routinemäßig für ähnliche oder andere Gesundheitszwecke od. dgl. verwandt werden, erfolgen.

Die Menge an biologisch aktivem Zusatz, d.h. z.B. Protein oder Hapten, als Antigen, die benötigt wird, hängt ab von:
- Zusammensetzung des Futters oder der Nahrung,
- Spezies,
- Alter,
- Geschlecht und
- Zucht behandelter Individuen oder Populationen,
- Dosis und Dauer der Immunisierung.

Als Richtlinie sei folgende Erfahrungs-Dosis angegeben:
1 g Eiweiß In 100 g flüssigem Futter für die intragastrale Stimulation.

Das Vorhandensein von Antikörpern aus Immunisierungen ist Voraussetzung für die Wirksamkeit der intragastralen Antigenapplikation.

### Anwendung der Erfindung

Optimale Ergebnisse werden erzielt bei der Mast von Tieren zur Fleisch-Erzeugung, nämlich Verkürzung der Mastdauer bei geringerem Futterverbrauch bis zum Erreichen des Schlachtendgewichts.

Zusätzlich fördert dieser immun-biologisch wirksame, wachstumsfördernde Effekt die Resistenzmechanismen im Körper, insbesondere bei Streß, durch spezifische und unspezifisch wirkende Abwehrsysteme peripher lymphatischer Organe und zirkulierender Lymphozyten in Blut und Lymphe.

### Versuch 1 (Pilotversuch)

In diesem Vorversuch wurden praxisbezogen in einem Schweinemastbetrieb Ferkel nach Absetzen von den Muttertieren mit Molkeeiweiß immunisiert. Danach erhielten die Tiere Molke im Trinkwasser zugesetzt.

Obwohl alle Tiere (n=20) an einer infektiösen Darmerkrankung litten, wurde das Wachstum der Tiere nur in den 10 unbehandelten Kontrolltieren durch die Infektion behindert. Nach Abklingen der Infektion setzte bei den behandelten Tieren eine noch höhere Wachstumsgeschwindigkeit ein.

Die tägliche Gewichtszunahme bis zum Schlachtgewicht betrug 675 g in der behandelten Gruppe und 600 g in der unbehandelten Gruppe.

Die Futterverwertung, d.h. die Menge Futter/kg, die zur Steigerung des Körpergewichts um 1 kg benötigt wurde, war 1 : 2,45 in der behandelten Gruppe gegenüber 1 : 3,25 bei den Kontrollen.

In dieser Pilotstudie verbesserte sich die tägliche Gewichtszunahme um + 12,5 % und die Futterverwertung um + 24 %.

Diese gemachten Erfahrungen und Beobachtungen bildeten die Grundlage für eine zweite Versuchsserie.

### Versuch 2 (Doppelblindversuch)

- Dauer:: 25 Wochen
- Versuchstiere:: 44 Schweine: deutsches veredeltes Landschwein, 22 männliche und 22 weibliche Tiere, als Ferkel in 4 Gruppen à 11 Tiere aufgeteilt.
- Vorbehandlung und Fütterung:: Im Alter von 28 Tagen wurden die Ferkel von den Muttertieren abgesetzt. Bis zur 10. Woche erhielten die Tiere handeisübliches, standardisiertes Ferkelaufzuchtfutter.
Nach Absetzen wurden die Tiere einer der folgenden Gruppen zufällig zugeteilt:
- Gruppe 1:: parenterale Sensibilisierung mit Molke und Einzelwiegen
- Gruppe 2:: parenterale Sensibilisierung mit Molke und Bestimmung des Gruppengewichts bei Beginn und Ende des Versuchs
- Gruppe 3:: keine Immunisierung (Kochsalzinjektion) und Einzelwiegen
- Gruppe 4:: keine Immunisierung (Kochsalzinjektion) und Bestimmung des Gruppengewichts bei Beginn und Ende des Versuchs.

### Sensibilisierung:

Die Tiere der Gruppe 1 und 2 wurden 3 x Immunisiert, Im Alter von 4, 7 und 10 Wochen, durch s.c. Injektion von 1,0 ml kommerzielle, konzentrierte Milch-Protein-Lösung (Molke, Proteingehalt 10 %).

Die Tiere erhielten von der 10. Woche an ein herkömmliches, einheitliches Mastleistungsfutter und zusätzlich Molke im Trinkwasser. Damit war eine kontinuierliche enterogastrale Stimulation zwischen dem oral aufgenommenen Milchprotein und der parenteral sensibilisierten Magenschleimhaut gesichert.

### Durchführung:

Jeweils 11 Schweine wurden in einem Verschlag auf Vollspaltenboden ohne Einstreu mit 2 x täglicher Flüssigfütterung (Molke und Mastfutter) und freiem Trinken (Molke im Trinkwasser) gehalten. Die verbrauchte Futtermenge und Molkemenge wurden für die jeweiligen Boxen elektronisch ermittelt und aufgezeichnet.

### Wiegen der Tiere:

Alle Tiere wurden zu Versuchsbeginn, nach 10 Wochen und bei Versuchsende nach 15 Wochen als Gruppe gewogen. In Gruppe 1 und 3 wurden alle Tiere zusätzlich einzeln gewogen, jeweils am Ende der 3., 5., 7. und 11. Woche der Mast. Das durchschnittliche Gewicht / Tier und Gruppe wurde bestimmt.

### Schlachtung und Bestimmung der Fleischqualität:

Alle Tiere wurden nach 15 Wochen (im Alter von ca. 25 Wochen) geschlachtet und das Fleisch entsprechend den EG-Richtlinien Klassifiziert.

### Ergebnisse:

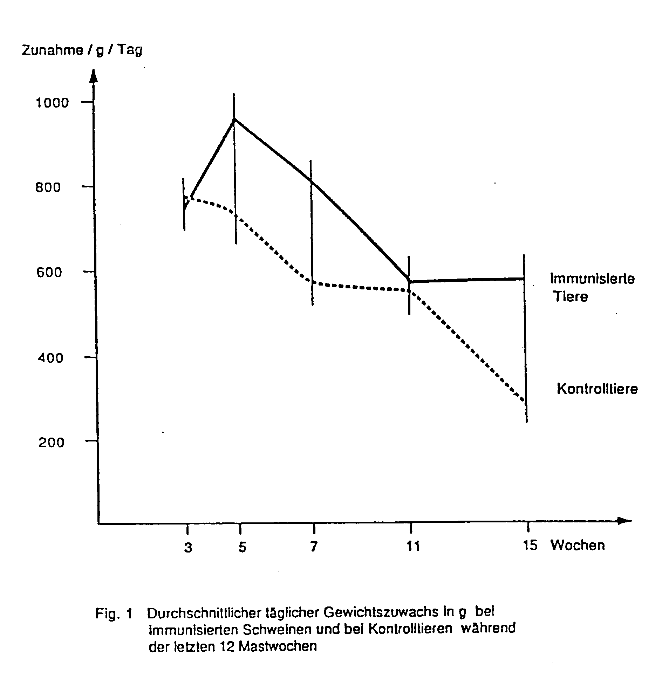

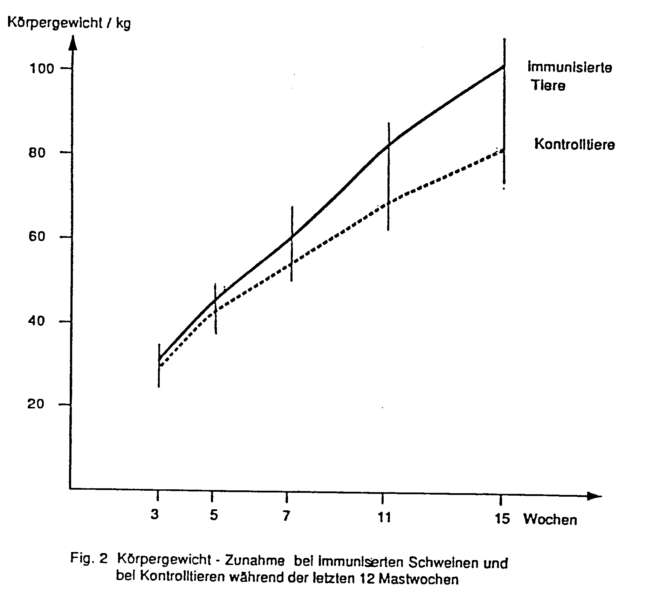

Tägliche Gewichtszunahme, Gesamtkörpergewicht am Ende des Versuchs, Gewichte und Futternutzungsraten aller Gruppen sind dargestellt in **Tabelle 1** und **Fig. 1** und **2** .

Die Schlachtkörperbeurteilung aller Tiere unabhängig von der Behandlung ergab nach den EG-Richtlinien die Klassifizierung E-1 (exzellent).

Die Ergebnisse des Versuchs 2 zeigen deutlich, daß nach vorausgegangener Immunisierung und nachfolgender enterogastraler Antigengabe die Futternutzung und damit die tägliche Gewichtszunahme sich erheblich steigern. Folglich sind Lebend- und Schlachtgewicht immunisierter Tiere nach 15 Wochen signifikant um + 16 % bzw. + 18,5 % gesteigert.

Diese Ergebnisse entsprechen jenen des Versuchs 1 und zeigen klar,
daß die Behandlung, d.h. Immunisierung und (nachfolgende) Applikation des Antigens mit Futter bei Tieren unter sonst gleichen Bedingungen,
nicht nur höchst wirksam ist mit um 30,4 % gesteigerter Futternutzung,
sondern auch äußerst wirtschaftlich vorteilhaft für den Mäster, da der Futterverbrauch für die Mast deutlich um 13,5 % sinkt.

Es wurden keine Nebenwirkungen mit dieser Behandlung gesehen.

Im Gegenteil, die Schweine in Gruppe 1 und 2 erholten sich von einer Virusinfektion, die Durchfall hervorrief, schneller als die Kontrolltiere; die tägliche Gewichtszunahme stieg nach überstandener Infektion sofort wieder an (Fig. 2), nicht jedoch in der Kontrollgruppe.

Diese Ergebnisse und Untersuchungen, die unter Feldbedingungen in beiden Versuchen gemacht wurden, beweisen deutlich, daß die Erfindung - basierend auf Untersuchungen an Labortieren - erfolgreich in der Tierproduktion angewendet werden kann.

### Literatur:

- ¹: Grossman, M.I. (1981):
Regulation of gastric acid secretion
In: Physiology of the Gastrointestinal Tract
Ed.: L.R. Johnson. Raven Press, New York, 659 - 671
- ²: Chey, W.Y., Lee, Y.H. (1978):
Plasma secretin concentration in fasting and postprandial state in man Digest. Dis. 23, 981

**Tabelle 1**

| Gewichtszunahme und Futternutzung bei mit Molke immunisierten und Kontroll-Tieren (Schweinen) während 15-wöchiger Mast | | | | | | |
|---|---|---|---|---|---|---|
| Gruppen & Behandlung | n | Gewichtszunahme/Tag (g) | Futternutzung | Futterverbrauch (kg) | nach 15 Wo. Lebendgewicht(kg) | Schlachtgewicht(kg) |
| 1 + 2 Immunisiert mit Molke | 22 | 604 | 2,75 | 175,0 | 97,7^{a} | 81,2^{a} |
| 3 + 4 Kontrolle | 22 | 530 | 3,95 | 202,4 | 84,2^{b} | 68,5^{b} |
| Differenz (%) 1+2 vs. 3+4 | | 74 | 1,20 | 27,4 | 13,5 | 12,7 |
| | | (+14) | (+30,4) | (-13,5) | (+16,0) | (+18,5) |

| | | | | | | |
|---|---|---|---|---|---|---|
| a vs. b: p < 0,05 (Wilcoxon-Test für nicht-parametrische Verteilungen) | | | | | | |
| b mangels Unterschieden Innerhalb jeweils der Gruppen 1 + 2 bzw. 3 + 4 sind die Ergebnisse zusammengefaßt | | | | | | |

## Patentansprüche

1. Verfahren zur Steigerung von Wachstumsgeschwindigkeit und/oder Nutzung von Futtermitteln und/oder Resistenz bei Tieren, ausgenommen zur Prophylaxe und Therapie von Störungen und Krankheiten im Verdauungstrakt, gekennzeichnet durch vorausgegangene Immunisierung bzw. Sensibilisierung mit nachfolgender und ggf. auch vorheriger und/oder gleichzeitiger gastrointestinaler Applikation des Antigens (der Antigene), mit folgenden Ausnahmen:
(a) ein Verfahren zur Steigerung der Wachstumsgeschwindigkeit bei Geflügel, das mit einem Futter großgezogen wird, das zugesetzte, lebensfähige, sporulierte Oozysten wenigstens einer Kokzidienart enthält, für die das Geflügel anfällig ist, wobei die Oozysten in einer Konzentration vorhanden sind, die nur ausreichend ist, um eine subklinische Infektion im Geflügel zu induzieren;
b) die Verwendung einer Zusammensetzung zur oralen Immunisierung und dadurch bedingten Wachstumssteigerung von Tieren, wie etwa Geflügel, die feste Teilchen einer lipidkontinuierlichen Emulsion von wenigstens 1 Gew.-%, aber nicht mehr als 70 Gew.-% Wasser in Lipid mit einem Schmelzpunkt im Bereich von 30 bis 55°C umfaßt, wobei die Zusammensetzung pro Teilchen im Mittel wenigstens ein lebendes, abgekapseltes, parasitisches Protozoon enthält;
(c) ein Verfahren zur Wachstums- und Resistenzsteigerung bei Schweinen durch orale Immunisierung und weitere orale Gabe von hitzeinaktivierten Antigen-Zubereitungen von E. coli-Stämmen, welche zu Darmkrankheiten führen; und
(d) ein Verfahren zur Wachstums- und Resistenzsteigerung bei präruminanten Kälbern durch orale Immunisierung und weitere orale Gabe von hitze-inaktivierten Antigen-Zubereitungen von E. coli- und Salmonella-Stämmen, welche zu Darmkrankheiten führen.

2. Verfahren nach Anspruch 1, dadurch gekennzeichnet, daß die Antigene tierischen, pflanzliche, viralen oder bakteriellen Ursprungs sind, insbesondere Aminosäuren, Peptide, Proteine, Skleroproteine, globuläre Proteine, Histone, Albumine, Globuline, Proteide, Nucleinsäuren, Nucleoside und Nucleotide, Prophyrine und Zellhämine, Lipide, Phospholipide, Glykolipide, Lipoproteine, Fettsäuren, Cerebroside und Ganglioside, Isoprenoidlipide, Steroide und Carotinoide, Polyprenole, Polyprenylchinone, Monosaccharide, Glykoside, Oligosaccharide und Polysaccharide, Glykogen, Komoglykane, Heteroglykane und Glykoproteine, Mikroorganismen und Protozoen.

3. Verfahren nach Anspruch 2, dadurch gekennzeichnet, daß als Antigene Bestandteile und/oder Produkte, insbesondere Stoffwechsel- oder Abbauprodukte der Antigene eingesetzt werden.

4. Verfahren nach Anspruch 1, dadurch gekennzeichnet, daß die Antigene synthetische Proteine sind, vorzugsweise Peptide, insbesondere Homopolymere, statische oder alternierende Copolymere, Blockpolymere, Propf-Copolymere, mit Antigen-Determinaten zum Auslösen einer Immunantwort.

5. Verfahren nach Anspruch 4, dadurch gekennzeichnet, daß die Antigene Haptene sind, insbesondere Nitrophenylessigsäure (NIT) und 2,4-Dinitrofluorobenzol (DNP).

6. Verfahren nach einem der Ansprüche 2 bis 5, dadurch gekennzeichnet, daß die Antigene als Haptene an ein Trägerprotein gekoppelt sind.

7. Verfahren nach einem der vorangehenden Ansprüche, dadurch gekennzeichnet, daß zur Immunisierung eine Menge von Antigen im Erzeugnis von 1 bis 500 mg/kg Körpergewicht eingesetzt wird.

8. Verfahren nach Anspruch 7, dadurch gekennzeichnet, daß zur Immunisiering das Antigen in einer Menge im Erzeugnis von 1 bis 100 mg/kg Körpergewicht ein- oder mehrfach parenteral appliziert wird.

9. Verfahren nach einem der vorangehenden Ansprüche, dadurch gekennzeichnet, daß das Antigen in einer Menge im Erzeugnis von 2 bis 500 mg/kg Körpergewicht je Tag enteral appliziert wird.

10. Verwendung von mindestens einem Antigen zum Gebrauch in einem Verfahren nach einem der Ansprüche 1 bis 9.

11. Verwendung von mindestens einem Antigen zur Herstellung eines Medikaments zur Steigerung von Wachstumsgeschwindigkeit und/oder Nutzung von Nahrungsmitteln und/oder Resistenz bei Menschen, wobei nach voausgegangener Immunisierung bzw. Sensibilisierung des Menschen durch das Antigen (die Antigene) nachfolgende und ggf. vorherige und/oder gleichzeitige gastrotestinale Applikation des Antigens (der Antigene) in geeigneter pharmazeutischer Formulierung erfolgt, ausgenommen für die Prophylaxe und Therapie von Störungen und Krankheiten im Verdauungstrakt.

## Claims

1. Method for increasing the rate of growth and/or utilization of feedstuffs and/or resistance in animals, except for the prophylaxis and treatment of disorders and diseases in the digestive tract, characterized by prior immunization or sensitization, with subsequent and, if appropriate, also previous and/or simultaneous gastrointestinal administration of the antigen (of the antigens), with the following exceptions:
(a) a method for increasing the rate of growth in poultry reared using a feed which contains added, viable,m sporulated oocysts of at least one coccidia species to which the poultry is susceptible, the oocysts being present in a concentration which is sufficient only to induce a subclinical infection in the poultry;
(b) the use of a composition for oral immunisation and, as a result of this, increased growth of animals such as, for example, poultry, which composition includes solid particles of a lipid-continuous emulsion of at least 1% by weight, but not more than 70% by weight, of water in lipid having a melting point in the range from 30 to 55°C, the composition containing on average at least one live, encapsulated, parasitic protozoon per particle;
(c) a method for increasing growth and resistance in pigs by oral immunization and additional oral administration of heat-inactivated antigen formulations of E. coli strains, which lead to intestinal diseases; and
(d) a method for increasing growth and resistance in preruminant calves by oral immunization and additional oral administration of heat-inactivated antigen formulations of E. coli and Salmonella strains, which lead to intestinal diseases.

2. Method according to Claim 1, characterised in that the antigens are of animals, vegetable, viral or baterial origin, in particular amino acids, peptides, proteins, scleroproteins, globular proteins, histones, albumins, globulins, proteids, nucleic acids, nucleosides and nucleotides, prophyrins and cytochromes, lipids, phospholids, glycolipids, lipoproteins, fatty acids, cerebrosides and gangliosides, isopranoid lipids, steroids and carotenoids, polyprenols, polyprenyl-quinones, monosaccharides, glycosides, oligosaccharides and polysaccharides, glycogen, homoglycans, heteroglycans and glycoproteins, microorganisms and protozoa.

3. Method according to Claim 2, characterised in that constituents and/or products, in particular metabolism or breakdown products, of the antigens are used as antigens.

4. Method according to Claim 1, characterized in that the antigens are synthetic proteins, preferably peptides, in particular homopolymers, random or alternating copolymers, block polymers, graft copolymers, with antigenic determinants for triggering an immune response.

5. Method according to Claim 4, characterized in that the antigens are haptens, in particular nitrophenylacetic acid (NIT)and 2,4-dinitrofluorobenzene (DNP).

6. Method according to one of Claims 2 to 5, characterized in that the antigens are coupled as haptens to a carrier protein.

7. Method according to one of the preceding claims, characterized in that, for immunization, a quantity of antigen of 1 to 500 mg/kg bodyweight is used in the product.

8. Method according to Claim 7, characterized in that, for immunization, the antigen is administered parenterally once or several times in a quantity of 1 to 100 mg/kg bodyweight in the product.

9. Method according to one of the preceding claim, charactarized in that the antigen is administered via the intestinal route in a quantity of 2 to 500 mg/kg bodyweight per day in the product.

10. Use of at least one antigen for application in a method according to one of Claims 1 to 9.

11. Use of at least one antigen for the preparation of a medicament for increasing the rate of growth and/or utilization of foodstuffs and/or resistance in humans, in which method, after prior immunization or sensitization of the human by the antigen (the antigens), subsequent and, if appropriate, previous and/or simultaneous gastrointestinal administration of the antigen (of the antigens) is carried out in a suitable pharmaceutical formulation, except for the prophylaxis and treatment of disorders and diseases in the digestive tract.

## Revendications

1. Procédé d'accroissement de la vitesse de croissance et/ou de l'utilisation de la nourriture et/ou de la résistance cher les animaux, à l'exception de la prophylaxie et due traitment des désordres et des maladies de l'appareil digestif, caractérisé par une immunisation ou sensibilisation préalable avec application gastro-intestinale ultérieure et le cas échéant également préalable et/ou simultanée due ou des antigène(s), avec les exceptions suivantes:
(a) un procédé d'accroissement de la vitesse de croissance de la volaille élevée avec une nourriture qui contient un ajout d'oocystes sporulés vivants appartenant à au moins une espèce de coccidie à laquelle la volaille est sensible, les oocystes étant présents à une concentration qui suffit simplement pour induire une infection sub-clinique chez la volaille,
(b) l'application d'une composition pour l'immunisation orale et l'augmentation de croissance des animaux, comme par exemple la volaille, ainsi provoquée, qui comprend des particules solides d'une émulsion à phase lipidique continue d'au moins 1% en poids, mais pas plus de 70% en poids, d'eau dans le lipide avec un point de fusion compris entre 30 et 55°C, où la composition contient par particule en moyenne au moins un protozoaire parasite vivant encapsulé;
(c) un procédé d'accroissement de la croissance et de le résistance chez les porcs par immunisation orale et administration orale ultérieure de préparations d'antigènes de souches d'E. coli conduisant à des maladies intestinales, inactivées à la chaleur; et
(d) un procédé d'accroissement de la croissance et de la résistance chez les veaux pré-ruminants et d'administration orale ultérieure de préparations d'antigènes de souches d'E. coli et de Salmonella conduisant à des maladies intestinales, inactivées à la chaleur.

2. Procédé selon la revendication 1, caractérisé en ce que les antigènes sont d'origine animale, végétale, virale ou bactérienne, en particulier des acides aminés, des peptides, des protéines, des scléroprotéines, des protéines globulaires, des histones, des albumines, des globulines, des protéides, des acides nucléiques, des nucléosides et des nucléotides, des prophyrines et des hémines cellulaires, des lipides, des phospholipides, des glycolipides, des lipoprotéines, des acides gras, des cérébrosides et des gangliosides, des lipides isoprénoïdes, des stéroïdes et des carotinoïdes, des polyprénols, des polyprénylquinones, des monosaccharides, des glycosides, des oligosaccharides et des polysaccharides, du glycogène, des homoglycanes, des héréroglycanes et des glycoprotéines, des microorganismes et des protozoaires:

3. Procédé solon la revendication 2, caractérisé en ce qu'on utilise comme antigènes des composants et/ou produits, en particulier des produits métaboliques ou de dégradation, des antigènes.

4. Procédé selon la revendication 1, caractérisé en ce que les antigènes sont don protéines de synthèse, de préférence des peptides, an particulier des homopolymères, des copolymères statiques ou alternants, des polymères séquencés, des copolymères greffés, avec des déterminants antigèniques pour déclencher une réponse immunitaire.

5. Procédé selon la revendication 4, caractérisé en ce que les antigènes sont des haptènes, en particulier l'acide nitropénylacétique (NIT) et le 2,4-dinitrofluorobenzène (DNP).

6. Procédé selon l'une des revendications 2 à 5, caractérisé en ce que les antigènes sont couplés en tant qu'haptènes à une protéine support.

7. Procédé selon l'une des revendication précédentes, caractérisé en ce qu'on utilise pour l'immunisation une quantité d'antigène dans le produit de 1 à 500 mg/kg de poids corporel.

8. Procédé selon la revendication 7, caractérisé en ce qu'on applique pour l'immunisation l'antigène en une qunatité dans le produit de 1 à 100 mg/kg de poids corporel, à une ou plusieurs reprises par voie parentérale.

9. Procédé selon l'une des revendications précédentes, caractérisé en ce qu'on applique l'antigène par voie entérale en une quantité dans le produit de 2 à 500 mg/kg de poids corporel par jour.

10. Application d'au moins un antigène aux fins d'utilisation dans un procédé selon l'une des revendications 1 à 9.

11. Application d'au moins un antigène à la préparation d'un médicament pour accroître la vitesse de croissance et/ou l'utilisation de la nourriture et/ou la résistance chez l'homme, où après immunisation ou sensibilisation préalable de l'homme par le(s) antigène(s) et le cas échéant application gastrointestinale préalable et/ou simultanée de l'antigène ou des antigènes on procède à la mise sous une forme pharmaceutique appropriée, à l'exception de la prophylaxie et du traitement des désordres et des maladies de l'appareil digestif.
